# EUROPEAN PATENT APPLICATION

(11) **EP 4 566 541 A1**
(43) Date of publication of application: **11.06.2025**
(21) Application number: 23214889.0
(22) Date of filing: 07.12.2023
(51) Int. Cl.: A61B 6/03, A61B 5/00, A61B 6/00, A61B 6/50, A61B 8/12, A61B 8/00, A61B 8/08, G06N 3/00

(54) **PREDICTING VESSEL CHARACTERISTICS**

(71) Applicant: Koninklijke Philips N.V., 5656 AG Eindhoven (NL)
(72) Inventor: LUCASSEN, Gerhardus Wilhelmus, Eindhoven (NL); LAI, Marco, Eindhoven (NL); HENDRIKS, Bernardus Hendrikus Wilhelmus, 5656AG Eindhoven (NL); SPLIETHOFF, Jarich Willem, Eindhoven (NL); SIO, Charles Frederik, Eindhoven (NL); GROEN, Joanneke, Eindhoven (NL); LARUE, Ruben, Eindhoven (NL); FERNANDO, Shakith Devinda, Eindhoven (NL)
(74) Representative: Philips Intellectual Property & Standards

(57) **Abstract**

A computer-implemented method of predicting vessel characteristics, is provided. The method includes: inputting an extracorporeal image (110) representing a vessel into a neural network (130); generating one or more predicted vessel characteristics (140) for the vessel (120) using the neural network (130) in response to the inputting; and outputting the one or more predicted vessel characteristics (140). The neural network (130) is trained to generate the one or more predicted vessel characteristics (140) from the inputted extracorporeal image (110) using training data comprising a plurality of extracorporeal training images (150) representing the vessel (120), and for each extracorporeal training image, an intravascular training image (160) representing the vessel (120), and ground truth data corresponding to the training data, the ground truth data representing the one or more vessel characteristics (140).

## Description

### TECHNICAL FIELD

The present disclosure relates to predicting vessel characteristics. A computer-implemented method, a computer program product, and a system, are disclosed.

### BACKGROUND

Vascular investigations are performed using a variety of imaging modalities. These include "extracorporeal" imaging modalities, and also intravascular imaging modalities. Examples of extracorporeal imaging modalities include (coronary) computed tomography "CT", coronary computed tomography angiography "CCTA", magnetic resonance imaging "MRI", positron emission tomography "PET", ultrasound "US" imaging, X-ray fluoroscopy, and X-ray angiography. Examples of intravascular imaging modalities include intravascular ultrasound "IVUS", optical coherence tomography "OCT", and intravascular photoacoustic "IVPA". Such imaging modalities are used to investigate vascular conditions such as coronary artery disease "CAD".

Extracorporeal imaging modalities are often used at a so-called pre-interventional procedure stage to diagnose the presence of a vascular condition. For instance, an investigation for CAD may be performed using CCTA images of the coronary arteries. The CCTA images may be used to determine the presence of a vessel lesion, and if present, to determine vessel characteristics relating to the lesion, such as a composition, and a morphology of a plaque in a vessel. The vessel characteristics that are obtained from the CCTA images may be used to assess the need for a subsequent intervention, for example by evaluating risk factors such as the risk of plaque rupture.

If an interventional procedure is deemed necessary, an intravascular imaging modality is often used to determine additional, or more accurate, vessel characteristics relating to the lesion. For instance, in an investigation for the presence of CAD, IVUS, or OCT, or IVPA images may be used to determine vessel characteristics such as estimates of lumen diameter, vessel diameter, luminal stenosis percentage, percentage plaque burden, calcium thickness, calcium distribution (e.g. luminal versus medial versus deep), a thickness of the so-called fibrous cap that covers plaque, an indication of the presence of thrombi, an indication of a stent reference landing zone (i.e. start and end locations for placing a stent for treating the lesion), and an indication of the stent geometry (e.g. stent diameter, and length). Such intravascular imaging modalities may be used at an interventional procedure planning stage, and also during an interventional procedure.

Owing to their non-invasive nature, the use of extracorporeal imaging modalities to determine vessel characteristics is preferred at the pre-interventional procedure stage of a vascular investigation. However, extracorporeal imaging modalities typically suffer from inferior image resolution, and inferior image quality, as compared to intravascular imaging modalities. For instance, extracorporeal CT images can suffer from blooming; an image artifact in which the relatively high density of calcium deposits in blood vessels causes them to appear larger than their true size. This can lead to an over-estimation of the calcium thickness in extracorporeal CT images. The thickness of the thin fibrous cap that covers plaque; a useful factor in assessing the risk of the plaque's vulnerability to rupture; is also hardly visible in using extracorporeal CT images. Thrombus detection can also be challenging using extracorporeal CT images. Drawbacks such as these, hamper the accuracy of diagnoses that are obtained from investigations that are performed using images generated by extracorporeal imaging modalities.

On the other hand, the vessel characteristics that are obtained using images that are generated by extracorporeal imaging modalities, can complement the vessel characteristics that are obtained using intravascular imaging modalities. For instance, the length of a lesion is a useful parameter in assessing the need for a subsequent intervention. An accurate measurement of the lesion length can be obtained from extracorporeal CT images. By contrast, the lesion length is difficult to measure accurately using IVUS images. Extracorporeal CT images can also provide more detailed depth information on calcium deposits than IVUS or OCT images.

Thus, there is a need to improve the assessment of vessel characteristics from images that are acquired using extracorporeal imaging modalities.

### SUMMARY

According to one aspect of the present disclosure, a computer-implemented method of predicting vessel characteristics, is provided. The method includes:
receiving angiographic data comprising an extracorporeal image representing a vessel;
inputting the extracorporeal image into a neural network;
generating one or more predicted vessel characteristics for the vessel using the neural network in response to the inputting; and
outputting the one or more predicted vessel characteristics; and
wherein the neural network is trained to generate the one or more predicted vessel characteristics from the inputted extracorporeal image using training data comprising a plurality of extracorporeal training images representing the vessel, and for each extracorporeal training image, an intravascular training image representing the vessel, and ground truth data corresponding to the training data, the ground truth data representing the one or more vessel characteristics.

Since the above method predicts vessel characteristics from an extracorporeal image, i.e. from an image that is acquired using a non-invasive imaging modality, the method may be used to support a physician in planning a vascular intervention without the need to acquire images of the vessel using an invasive imaging modality. Moreover, since the method predicts the vessel characteristics using a neural network that is trained using both extracorporeal training images and intravascular training images, the vessel characteristics predicted by the neural network include characteristics that may not be obtained from an extracorporeal image alone, or that may not be obtained as accurately, as from an extracorporeal image alone. Thus, the method helps to bridge the gap between the capabilities of extracorporeal imaging modalities and intravascular imaging modalities, without the need to acquire images of the vessel using an invasive imaging modality.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a computer-implemented method of predicting vessel characteristics, in accordance with some aspects of the present disclosure.
Fig. 2 is a schematic diagram illustrating an example of a system 200 for predicting vessel characteristics, in accordance with some aspects of the present disclosure.
Fig. 3 is a schematic diagram illustrating an example of various elements of a method of predicting vessel characteristics, in accordance with some aspects of the present disclosure.
Fig. 4 is a schematic diagram illustrating a first example of various elements of a method of training a neural network to generate one or more predicted vessel characteristics, in accordance with some aspects of the present disclosure.
Fig. 5 is a schematic diagram illustrating a second example of various elements of a method of training a neural network to generate one or more predicted vessel characteristics, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a computer-implemented method of predicting vessel characteristics. In some examples, reference is made to the vessel being a coronary artery, i.e. a vessel within the heart. However, it is noted that the computer-implemented method of predicting vessel characteristics is not limited to this example. In general, the vessel may be any type of blood vessel within the anatomy, and the blood vessel may be in any region of the anatomy. For instance, the vessel may be an artery, or a vein, for example, and the vessel may be in the cardiac region, or in the lung, the leg, the arm, the brain, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are performed in the computer-implemented methods disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations performed in the computer-implemented methods disclosed herein.

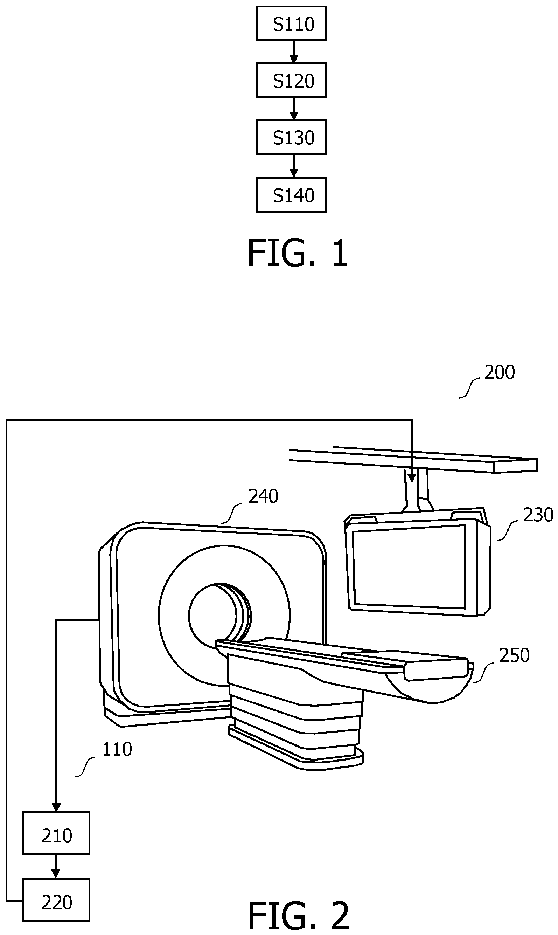

As mentioned above, there is a need to improve the assessment of vessel characteristics from images that are acquired using extracorporeal imaging modalities. Fig. 1 is a flowchart illustrating an example of a computer-implemented method of predicting vessel characteristics, in accordance with some aspects of the present disclosure. Fig. 2 is a schematic diagram illustrating an example of a system 200 for predicting vessel characteristics, in accordance with some aspects of the present disclosure. It is noted that operations described in relation to the method illustrated in Fig. 1, may also be performed by the one or more processors 220 of the system 200 illustrated in Fig. 2. Likewise, operations described as being performed by the one or more processors 220 of the system 200 illustrated in Fig. 2, may also be performed in the method described with reference to Fig. 1. With reference to Fig. 1, and Fig. 2, the computer-implemented method of predicting vessel characteristics, comprises:
receiving S110 angiographic data comprising an extracorporeal image 110 representing a vessel 120;
inputting S120 the extracorporeal image 110 into a neural network 130;
generating S130 one or more predicted vessel characteristics 140 for the vessel 120 using the neural network 130 in response to the inputting; and
outputting S140 the one or more predicted vessel characteristics 140; and
wherein the neural network 130 is trained to generate the one or more predicted vessel characteristics 140 from the inputted extracorporeal image 110 using training data comprising a plurality of extracorporeal training images 150 representing the vessel 120, and for each extracorporeal training image, an intravascular training image 160 representing the vessel 120, and ground truth data corresponding to the training data, the ground truth data representing the one or more vessel characteristics 140.

Since the above method predicts vessel characteristics from an extracorporeal image, i.e. from an image that is acquired using a non-invasive imaging modality, the method may be used to support a physician in planning a vascular intervention without the need to acquire images of the vessel using an invasive imaging modality. Moreover, since the method predicts the vessel characteristics using a neural network that is trained using both extracorporeal training images and intravascular training images, the vessel characteristics predicted by the neural network include characteristics that may not be obtained from an extracorporeal image alone, or that may not be obtained as accurately, as from an extracorporeal image alone. Thus, the method helps to bridge the gap between the capabilities of extracorporeal imaging modalities and intravascular imaging modalities, without the need to acquire images of the vessel using an invasive imaging modality.

The operations that are performed in the method illustrated in Fig. 1, are described in more detail below.

In the operation S110, angiographic data is received. The angiographic data comprises an extracorporeal image 110 representing a vessel 120.

The angiographic data that is received in the operation S 110 may be generated by various types of medical imaging systems. These include two-dimensional "2D" medical imaging systems such as projection X-ray imaging systems and 2D ultrasound imaging systems, and also three-dimensional, i.e. "volumetric" medical imaging systems such as computed tomography "CT" imaging systems, magnetic resonance imaging "MRI" systems, 3D ultrasound imaging systems, positron emission tomography "PET" imaging systems, and single photon emission computed tomography "SPECT" imaging systems. In examples in which the angiographic data is generated by a projection X-ray imaging system, or by a CT imaging system, the imaging system may be a spectral X-ray projection imaging system, or a spectral CT imaging system, respectively. Such imaging systems generate angiographic data representing X-ray attenuation within multiple different energy intervals. The angiographic data generated by such imaging systems may be processed, e.g. using various material decomposition algorithms, in order to distinguish between media that have similar X-ray attenuation values when measured within a single energy interval, and which would be indistinguishable in angiographic data generated by conventional projection X-ray imaging systems, or conventional CT imaging systems. Thus, angiographic data generated by spectral X-ray projection imaging systems, or by spectral CT imaging systems, may be processed to provide extracorporeal images with improved specificity to materials such as contrast agent, tissue, bone, the constituents of plaque, and so forth.

With continued reference to the operation S110, in some examples, the angiographic data is generated subsequent to the injection of a contrast agent into the vasculature of a subject. The contrast agent serves to improve the contrast between blood and surrounding tissue in the extracorporeal image 110. In these examples, the extracorporeal image 110 may be referred-to as contrast-enhanced extracorporeal image. Contrast-enhanced extracorporeal images may be generated by a projection X-ray imaging system, or by a CT imaging system, or by an MRI system. Extracorporeal MRI images may alternatively be generated in the absence of a contrast agent, and are often referred-to as non-contrast-enhanced MR angiography "NC-MRA" images.

The angiographic data comprises an extracorporeal image 110 representing a vessel 120. The vessel 120 may be a coronary artery, for example, i.e. a vessel within the heart. However, more generally, the vessel represented in the extracorporeal image may be any type of blood vessel within the anatomy, and the blood vessel may be in any region of the anatomy. For instance, the vessel may be an artery, or a vein, for example, and the vessel may be in the cardiac region, or in the lung, the leg, the arm, the brain, and so forth.

With continued reference to the operation S110, in one example, the extracorporeal image 110 is generated prior to performing an interventional procedure on the vessel 120. In this example, the extracorporeal image 110 may be referred-to as a pre-interventional procedure image. The extracorporeal image 110 may have been generated some seconds, minutes, hours, or even days, or a longer period, before performing the interventional procedure on the vessel 120. In another example, the extracorporeal image 110 be generated during an interventional procedure on the vessel 120. In this example, the extracorporeal image 110 may be referred-to as an intra-interventional procedure image.

With continued reference to the operation S110, the angiographic data may be received from various sources in the operation S110. For instance, the angiographic data may be received from a medical imaging system such as one of the example medical imaging systems described above. Alternatively, the angiographic data may be received from another source, such as from a computer readable storage medium, or from the internet, or from the Cloud, and so forth. In general, the angiographic data may be received via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

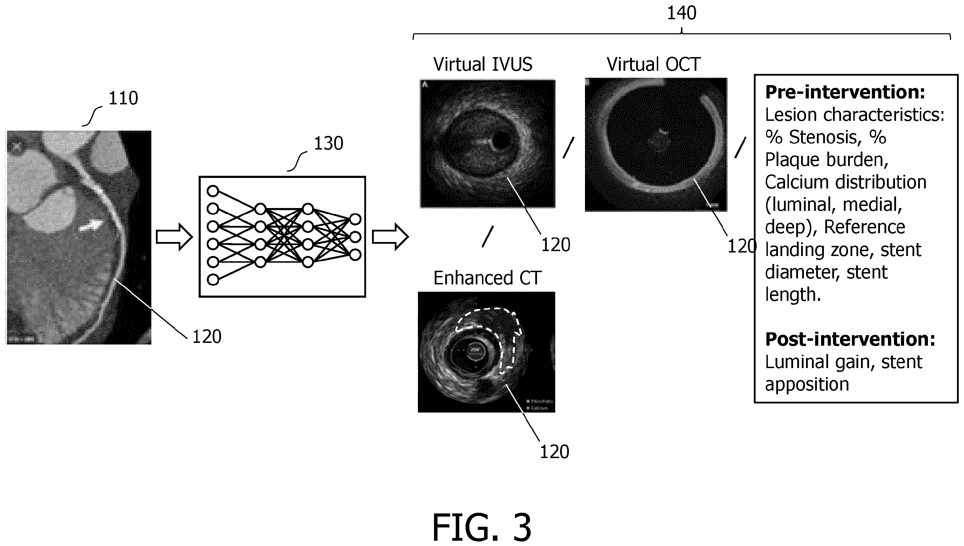

An example of the angiographic data that may be received in the operation S110 is illustrated in Fig. 3, which is a schematic diagram illustrating an example of various elements of a method of predicting vessel characteristics, in accordance with some aspects of the present disclosure. In the example illustrated in Fig. 3, the angiographic data comprises a CT image 110 representing a coronary artery 120.

Returning to the method illustrated in Fig. 1; in the operation S120, the extracorporeal image 110 is inputted into a neural network 130. In the related operation S130, one or more predicted vessel characteristics 140 for the vessel 120 are generated using the neural network 130 in response to the inputting. The neural network 130 is trained to generate the one or more predicted vessel characteristics 140 from the inputted extracorporeal image 110 using training data comprising a plurality of extracorporeal training images 150 representing the vessel 120, and for each extracorporeal training image, an intravascular training image 160 representing the vessel 120, and ground truth data corresponding to the training data, the ground truth data representing the one or more vessel characteristics 140.

Examples of predicted vessel characteristics 140 that may be generated by the neural network 130 in the operation S130 include vessel characteristics such as: a composition of a plaque in the vessel, a morphology of the plaque in the vessel, the vessel diameter, the vessel lumen diameter, luminal stenosis percentage, percentage plaque burden, calcium thickness, calcium distribution (e.g. luminal versus medial versus deep), a thickness of the so-called fibrous cap that covers plaque, an indication of the presence of thrombi, an indication of a stent reference landing zone (i.e. start and end locations for placing a stent for treating a lesion), an indication of the stent geometry (e.g. stent diameter, and length). Such vessel characteristics relate to a vessel lesion, and may therefore be referred-to as vessel lesion characteristics. Such vessel characteristics may also relate to a state of the vessel prior to an interventional procedure, and may also be referred-to as pre-interventional procedure vessel lesion characteristics. Other examples of predicted vessel characteristics 140 that may be generated by the neural network 130 in the operation S130 include luminal gain, and stent apposition. Such vessel characteristics result from performing an interventional procedure on the vessel lesion, and may therefore be referred-to as post-interventional procedure vessel characteristics.

In one example, the vessel characteristic(s) are predicted in the form of a list that includes an identification of the characteristic(s) and its relevant value. In another example, the vessel characteristic(s) are predicted in the form of a graphical representation of the vessel 120, and the one or more characteristics 140, such as those mentioned above, are annotated on the graphical representation of the vessel. The graphical representation of the vessel may for instance be a virtual IVUS image, or a virtual OCT image, or an enhanced CT image. Examples of these characteristics, and also their prediction in the form of a list, and graphical representations, are illustrated on the right-hand side of Fig. 3.

The neural network 130 that generates the one or more predicted vessel characteristics 140 in the operation S 130 may be provided by various architectures. These include a convolutional neural network "CNN", a recurrent neural network "RNN", and a transformer, for example. In general, the training of the neural network 130 involves inputting the training data into the neural network, and iteratively adjusting the neural network's parameters until the trained neural network provides an accurate output. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem, including gradient descent, Quasi-Newton methods, and so forth. Various algorithms have been developed to implement these methods and their variants, including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

The training of a neural network is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

In one example, the neural network 130 is trained to generate the one or more predicted vessel characteristics 140 by:
receiving the training data; and
for each of a plurality of the extracorporeal training images 150:
inputting the extracorporeal training image, and the corresponding intravascular training image 160, into the neural network 130;
generating one or more predicted vessel characteristics 140 for the vessel 120, using the neural network 130; and
adjusting parameters of the neural network 130 based on a difference between the one or more predicted vessel characteristics 140 and the corresponding one or more vessel characteristics from the ground truth data; and
repeating the inputting, the generating, and the adjusting, until a stopping criterion is met.

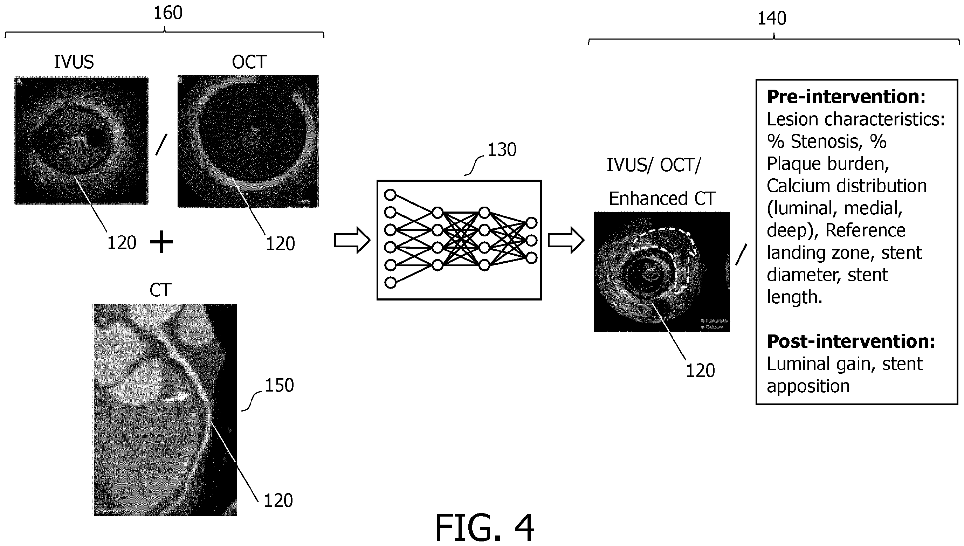

An example of the training of the neural network in accordance with this example is illustrated in Fig. 4, which is a schematic diagram illustrating a first example of various elements of a method of training a neural network to generate one or more predicted vessel characteristics, in accordance with some aspects of the present disclosure. As illustrated in the lower left-hand portion of Fig. 4, in this example the extracorporeal training images 150 representing the vessel 120 are CT images. As illustrated in the upper left-hand portion of Fig. 4, in this example the intravascular training images 160 are IVUS or OCT images. The use of IVPA intravascular training images 160 is also contemplated. The ground truth data corresponds to the training data. The ground truth data also corresponds to the intended output of the neural network. Thus, in the example illustrated in Fig. 4, the ground truth data may comprise a list of one or more values that represent the one or more predicted vessel characteristics 140 that are generated by the neural network 130. Examples of predicted vessel characteristics 140 that may be generated by the neural network 130 include pre-interventional procedure vessel lesion characteristics such as: luminal stenosis percentage, percentage plaque burden, calcium distribution (e.g. luminal versus medial versus deep), an indication of a stent reference landing zone (i.e. start and end locations for placing a stent for treating a lesion), an indication of the stent geometry (e.g. stent diameter, and length). Other examples of predicted vessel characteristics 140 that may be generated by the neural network 130 include post-interventional procedure vessel characteristics such as: luminal gain, and stent apposition. Alternatively, the ground truth data may comprise a graphical representation of the vessel 120, and wherein the one or more predicted vessel characteristics 140 that are generated by the neural network 130, are annotated on the graphical representation of the vessel.

In general, the training data that is used to train the neural network 130 may include extracorporeal training images 150 and corresponding intravascular training images 160 from some tens, hundreds, thousands, or more, different vessels. The training data may be obtained from vessels in subjects having a range of different ages, body mass index, co-morbidities, and so forth.

In a related example, the predicted vessel characteristics 140 are generated in the form of a graphical representation of the vessel 120. In this example, the locations of image artifacts are identified in the extracorporeal training images 150, and during training, the value of a cost function used to train the neural network is penalized based on a presence of an image artifact in the corresponding location in the graphical representation of the vessel 120 generated by the neural network. Penalizing the cost function in accordance with this example has the effect of reducing the image artifacts, and thereby improving image quality, in the graphical representation of the vessel 120 that is generated by the neural network. This example may be used to reduce image artifacts such as calcium blooming, for example.

In this example, for each of a plurality of the extracorporeal training images 150, the extracorporeal training image comprises an identification of a location of one or more image artifacts; and
wherein the neural network 130 is trained to generate the one or more predicted vessel characteristics 140 by outputting the one or more predicted vessel characteristics 140 in the form of a graphical representation of the vessel 120, and wherein the one or more characteristics are annotated on the graphical representation of the vessel 120; and
wherein the adjusting parameters of the neural network 130 is performed based on a value of a cost function, and wherein the value of the cost function is determined based on the difference between the one or more predicted vessel characteristics 140 and the corresponding one or more vessel characteristics from the ground truth data, and wherein the value of the cost function is penalized based on a presence of an image artifact in the corresponding location in the graphical representation of the vessel 120.

In one example, the ground truth data comprises annotations representing the one or more vessel characteristics, and the annotations are provided on the extracorporeal training images 150 and/or on the intravascular training images 160.

Providing the ground truth data in this manner may simplify the curation of the training data. The annotations may be provided manually, for example by a physician, or automatically, for example by using various automatic image analysis techniques.

In another example, and which may be used in combination with the above example of training the neural network, virtual training images are used to train the neural network 130. In this example, generator neural networks are used to generate virtual intravascular training image 160^{V} corresponding to each extracorporeal training image, and virtual extracorporeal training image 150^{V} corresponding to each intravascular training image. The virtual intravascular training images 160^{V}, and the virtual extracorporeal training images 150^{V}, are then used to train the neural network.

In this example, the neural network 130 is trained to generate the one or more predicted vessel characteristics 140 by:
inputting the extracorporeal training images 150 into a first generator neural network 170; and
generating a virtual intravascular training image 160^{V} corresponding to each extracorporeal training image using the first generator neural network 170 in response to the inputting;
wherein the first generator neural network 170 is trained to generate the virtual intravascular training images 160^{V} using second training data comprising a plurality of extracorporeal training images 150 representing the vessel 120, and for each extracorporeal training image, a corresponding intravascular training image 160 representing the vessel 120; and
inputting the intravascular training images 160 into a second generator neural network 180; and
generating a virtual extracorporeal training image 150^{V} corresponding to each intravascular training image using the second generator neural network 180 in response to the inputting;
wherein the second generator neural network 180 is trained to generate the virtual extracorporeal training images 150^{V} using third training data comprising a plurality of intravascular training images 160 representing the vessel 120, and for each intravascular training image, a corresponding extracorporeal training image 150 representing the vessel 120; and
training the neural network 130 to generate the one or more vessel characteristics 140 using the virtual intravascular training images 160^{V} and the virtual extracorporeal training images 150^{V}.

The training that is provided in accordance with this example has the effect of training the first generator neural network 170, the second generator neural network 180, and also the neural network 130. The use of virtual training images to train the neural network 130 in accordance with this example may be referred-to as cross-training of the first and second generator neural networks 170, 180. Advantageously, this technique of training the neural network 130 reduces the amount of training data that is required to train the neural network 130.

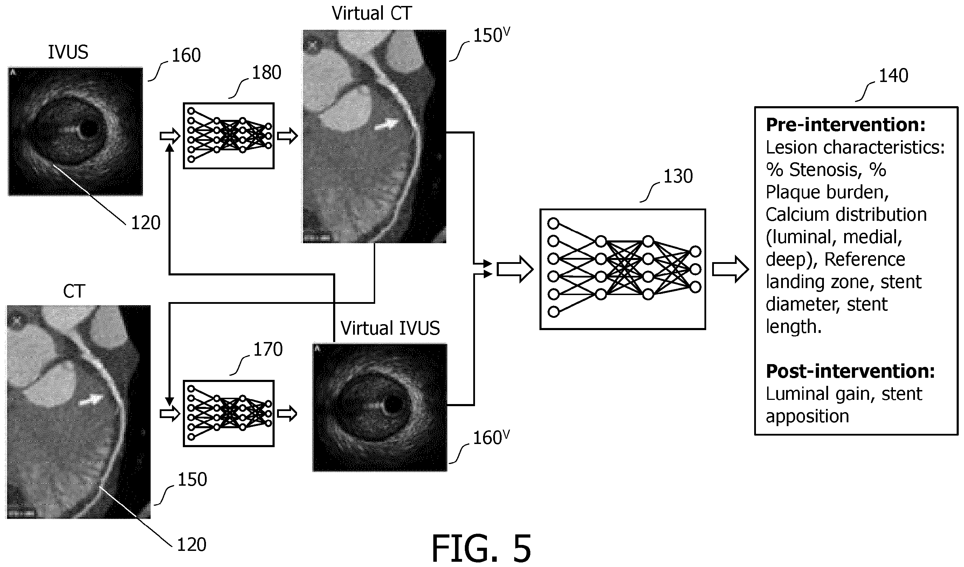

An example of the training of the neural network in accordance with this example is illustrated in Fig. 5, which a schematic diagram illustrating a second example of various elements of a method of training a neural network to generate one or more predicted vessel characteristics, in accordance with some aspects of the present disclosure. In this example, the first and second generator neural networks 170 and 180 may be provided by architectures such as Generative Adversarial Networks, i.e. "GANs". As illustrated in the lower left-hand portion of Fig. 5, in this example, the extracorporeal training images 150 representing the vessel 120 are CT images. As illustrated in the upper left-hand portion of Fig. 5, in this example, the intravascular training images 160 are IVUS images. The ground truth data corresponds to the training data. The ground truth data also corresponds to the intended output of the neural network. Thus, in the example illustrated in Fig. 5, the ground truth data may comprise a list of one or more values that represent the one or more predicted vessel characteristics 140 that are generated by the neural network 130. Examples of predicted vessel characteristics 140 that may be generated by the neural network 130 include pre-interventional procedure vessel lesion characteristics such as: luminal stenosis percentage, percentage plaque burden, calcium distribution (e.g. luminal versus medial versus deep), an indication of a stent reference landing zone (i.e. start and end locations for placing a stent for treating a lesion), an indication of the stent geometry (e.g. stent diameter, and length). Other examples of predicted vessel characteristics 140 that may be generated by the neural network 130 include post-interventional procedure vessel characteristics such as: luminal gain, and stent apposition. Alternatively, the ground truth data may comprise a graphical representation of the vessel 120, and wherein the one or more predicted vessel characteristics 140 that are generated by the neural network 130, are annotated on the graphical representation of the vessel.

In the example illustrated in Fig. 5, the extracorporeal CT training images 150 are inputted into the first generator neural network 170. In response, the first generator neural network 170 generates corresponding virtual intravascular training images 160^{V}. The intravascular training images 160 are inputted into the second generator neural network 180. In response, the second generator neural network 180 generates corresponding virtual extracorporeal training images 150^{V}. The virtual intravascular training images 160^{V}, and the virtual extracorporeal training images 150^{V}, are then used to train the neural network 130 to generate the one or more vessel characteristics 140 using the technique described above. In this example, the first generator neural network 170, the second generator neural network 180, and the neural network 130 may be trained simultaneously.

In a related example, the trained second generator neural network 180 is used to generate a virtual extracorporeal image 150^{V} from a post-interventional procedure intravascular image. In this example, the extracorporeal image 110 representing the vessel 120, which is inputted into the neural network 130 at inference, is a pre-interventional procedure image.

In this example, the method illustrated in Fig. 1 comprises:
receiving intravascular image data comprising a post-interventional procedure intravascular image representing the vessel 120;
inputting the post-interventional procedure intravascular image into the second generator neural network 180; and
generating a virtual extracorporeal image 150^{V} corresponding to the inputted post-interventional procedure intravascular image using the second generator neural network 180 in response to the inputting; and
outputting the virtual extracorporeal image 150^{V}.

The virtual extracorporeal image 150^{V} that is generated in accordance with this example is therefore a predicted post-interventional procedure virtual extracorporeal image 150^{V}, or in other words, a prediction of how the vessel would appear in the post-interventional procedure stage. The predicted post-interventional procedure virtual extracorporeal image 150^{V} facilitates an assessment of the procedure from an extracorporeal perspective without the need to acquire a real post-interventional procedure extracorporeal image, and consequently simplifies workflow.

In a related example, the method illustrated in Fig. 1 comprises:
comparing the virtual extracorporeal image 150^{V} with the inputted pre-interventional procedure extracorporeal image 110 representing the vessel 120; and
outputting a result of the comparing.

A physician may use the result of the comparing to assess the effectiveness of the procedure.

In any of the training examples described above, the training of the neural network 130 may be performed using intravascular training images 160 that are mutually registered to their corresponding extracorporeal training images 150. Thus, in one example, for each of a plurality of the intravascular training images, the intravascular training image 160 and the corresponding extracorporeal training image 150 are mutually registered.

Mutually registering the intravascular training images with the corresponding extracorporeal training images in accordance with this example can improve the accuracy of the predictions that are made by the neural network 130. An example of the mutual registration of the intravascular training images 160 with their corresponding extracorporeal training images 150 is described below in which the mutual registration includes registering the centerlines of the images. In this example, the intravascular training images 160 may be IVUS or OCT or IVPA images, and the extracorporeal training images 150 may be CT images. The IVUS/ OCT/ IVPA images represent transaxial slices through a plurality of different positions along a centerline of the vessel. A vessel centerline may be defined in each CT image and in each IVUS/ OCT/ IVPA by segmenting the vessel in each image and defining the vessel centerline as the geometric center of the vessel. The mutual registration of the intravascular training images with the corresponding extracorporeal training images may be performed by elastically deforming the centerline of the IVUS/ OCT/ IVPA images of the vessel such that the shape of the centerline in the IVUS/ OCT/ IVPA images of the vessel corresponds to the shape of the centerline of the vessel in the corresponding CT image. Alternatively, the mutual registration of the intravascular training images with the corresponding extracorporeal training images may be performed by elastically deforming the centerline of the CT image of the vessel such that the shape of the centerline in the CT image of the vessel corresponds to the shape of the centerline of the vessel in the corresponding IVUS/ OCT/ IVPA images. In this latter example, the shape of the centerline of the vessel in the IVUS/ OCT/ IVPA images may be a straight line.

In a related example, the operation of mutually registering the intravascular training images with the corresponding extracorporeal training images includes registering the centerlines of the intravascular training images with the centerlines of the corresponding extracorporeal training images, and also mutually registering image features that are common to both the intravascular training images and the corresponding extracorporeal training images. The image features may include features such as calcifications, vessel bifurcations, and so forth. In some cases, degraded image quality may result in an inaccurate, or an indeterminable, centerline position at some locations along a vessel. A more accurate registration may therefore be obtained performing the mutual registration by additionally using such image features.

Returning to the method illustrated in Fig. 1; in the operation S140, the one or more predicted vessel characteristics 140 are outputted. The one or more predicted vessel characteristics 140 may be outputted in various ways, including to a display device such as to the monitor 230 illustrated in Fig. 1, or to a virtual/ augmented reality display device, or to a computer-readable storage medium, or to the internet, or to the Cloud, and so forth.

In one example, the operation of outputting S 140 the one or more predicted vessel characteristics 140 comprises:
outputting the one or more predicted vessel characteristics 140 in the form of a list of one or more values, and wherein the one or more values represent the one or more characteristics.

In another example, the operation of outputting S140 the one or more predicted vessel characteristics 140 comprises :
outputting the one or more predicted vessel characteristics 140 in the form of a graphical representation of the vessel 120, and wherein the one or more characteristics 140 are annotated on the graphical representation of the vessel.

Examples of these forms of outputted predicted vessel characteristics 140 are illustrated on the right-hand side of Fig. 3. In the latter example, the graphical representation of the vessel 120 comprises a virtual extracorporeal image and/or a virtual intravascular image. The virtual intravascular image may be a virtual IVUS image, or a virtual OCT image, or a virtual IVPA image, for example. The virtual intravascular image may have a relatively higher image resolution than the extracorporeal image 110 which is inputted into the neural network 130 in the operation S120. The virtual extracorporeal image and/or a virtual intravascular image may be predicted by using corresponding types of images as the ground truth data. For instance, if it is intended that the neural network 130 generates predicted vessel characteristics 140 in the form of virtual extracorporeal CT image wherein the one or more characteristics 140 are annotated on the virtual extracorporeal CT image, then the ground truth data includes extracorporeal CT images with annotations of the vessel characteristics.

In one example, in addition to outputting the one or more predicted vessel characteristics 140, the method illustrated in Fig. 1 may also include outputting a graphical representation of the corresponding inputted extracorporeal image 110. By outputting a graphical representation of the corresponding inputted extracorporeal image 110, a physician may appreciate correspondences between the predicted vessel characteristics 140 that are generated by the neural network, and the inputted extracorporeal image 110, and thereby gain confidence in the method.

In a related example, the one or more predicted vessel characteristics 140 are outputted in the operation S140 in the form of a graphical representation of the vessel 120, and the outputted graphical representation of the corresponding inputted extracorporeal image 110 is registered to the graphical representation of the vessel 120. In another related example, the one or more predicted vessel characteristics 140 are outputted in the operation S140 in the form of a graphical representation of the vessel 120, and the outputted graphical representation of the corresponding inputted extracorporeal image 110 is overlaid, or displayed adjacent to the graphical representation of the vessel 120.

In one example, the neural network 130 is trained to generate a confidence measure for the one or more predicted vessel characteristics 140. In this example, the method illustrated in Fig. 1 comprises:
generating a value for the confidence measure in response to the inputting; and
outputting the value of the confidence measure.

In this example, the confidence measure may be evaluated by based on a measure of the quality of the inputted extracorporeal image 110. The image quality may be determined based on factors such as a measure of image resolution, a measure of signal-to-noise level, a measure of the image artifacts, and so forth. Alternatively, the confidence measure may be evaluated by adding noise to the inputted extracorporeal image 110 and determining the sensitivity of the outputted one or more predicted vessel characteristics 140 to the noise.

In one example, at inference, a point spread function of the extracorporeal image 110 is inputted into the neural network 130 and used to improve the accuracy of the predictions of the neural network 130, or to process a virtual extracorporeal image generated by the neural network, and to thereby improve the image quality of the virtual extracorporeal image. The sharpness of the virtual extracorporeal image may be improved, for example.

In this example, the method illustrated in Fig. 1 comprises receiving point spread function data representing a point spread function of the inputted extracorporeal image 110; and
i) inputting the point spread function data into the neural network 130; and generating one or more predicted vessel characteristics 140 for the vessel 120 based further on the point spread function data; and
   wherein the training data further comprises point spread function training data representing a point spread function of the extracorporeal training images 150;
   or
ii) wherein the outputting S140 the one or more predicted vessel characteristics 140 comprises outputting the one or more predicted vessel characteristics in the form of a virtual extracorporeal image of the vessel 120, and wherein the one or more characteristics are annotated on the virtual extracorporeal image of the vessel 120; and
   wherein the method further comprises processing the virtual extracorporeal image of the vessel 120 using the point spread function to improve an image quality of the virtual extracorporeal image.

The point spread function for the inputted extracorporeal image 110 may be measured using known techniques for the imaging system that generates the extracorporeal image 110. For example, it may be measured using the technique described in a document by Kayugawa, A., et al., "Accurate determination of CT point-spread-function with high precision", J. Appl. Clin. Med. Phys. 2013 Jul 8;14(4):3905. Alternatively, the point spread function for the inputted extracorporeal image 110 may be calculated from the image using known techniques.

In the first of these examples, associations between the point spread function and the inputted extracorporeal image 110 are used by the neural network 130 to generate more accurate predictions of the one or more predicted vessel characteristics 140. In the second of these examples, the virtual extracorporeal image of the vessel 120 which is generated by the neural network 130, is processed using the point spread function to improve an image quality of the virtual extracorporeal image. By way of an example, this processing may be performed using the deconvolution technique described in a document by Dougherty, D., et al., "The point spread function revisited: Image restoration using 2-D deconvolution", Radiography, 7(4):255-262. In the example of the inputted extracorporeal image 110 being generated by a spectral X-ray projection imaging system, or by a spectral CT imaging system, the point spread function may be applied separately for each of a plurality of different materials.

In another example, a computer program product is provided. The computer program product comprises instructions which when executed by one or more processors 210, cause the one or more processors to carry out a method of predicting vessel characteristics. The method comprises:
receiving S110 angiographic data comprising an extracorporeal image 110 representing a vessel 120;
inputting S120 the extracorporeal image 110 into a neural network 130;
generating S130 one or more predicted vessel characteristics 140 for the vessel 120 using the neural network 130 in response to the inputting; and
outputting S140 the one or more predicted vessel characteristics 140; and
wherein the neural network 130 is trained to generate the one or more predicted vessel characteristics 140 from the inputted extracorporeal image 110 using training data comprising a plurality of extracorporeal training images 150 representing the vessel 120, and for each extracorporeal training image, an intravascular training image 160 representing the vessel 120, and ground truth data corresponding to the training data, the ground truth data representing the one or more vessel characteristics 140.

In another example, a system 200 for predicting vessel characteristics, is provided. The system comprises:
an input unit 210;
one or more processors 220; and
an output unit 230;
wherein the input unit is configured to:
   receive S 110 angiographic data comprising an extracorporeal image 110 representing a vessel 120; and
   provide the angiographic data to the one or more processors 210;
   wherein the one or more processors 210 are configured to:
      implement a neural network 130;
      input S120 the extracorporeal image 110 into the neural network 130; and
      generate S 130 one or more predicted vessel characteristics 140 for the vessel 120 using the neural network 130 in response to the inputting; and
      wherein the output unit 220 is configured to output S 140 the one or more predicted vessel characteristics 140; and
      wherein the neural network 130 is trained to generate the one or more predicted vessel characteristics 140 from the inputted extracorporeal image 110 using training data comprising a plurality of extracorporeal training images 150 representing the vessel 120, and for each extracorporeal training image, an intravascular training image 160 representing the vessel 120, and ground truth data corresponding to the training data, the ground truth data representing the one or more vessel characteristics 140.

An example of the system 200 is illustrated in Fig. 2. In this example, the input unit 210 is provided by a memory that stores the angiographic data that is generated by the CT imaging system 240. In this example, the output unit 220 is provided by a monitor 230, and which is configured to display the one or more predicted vessel characteristics 140, and other outputs generated by the one or more processors 220. It is noted that the system 200 may also include one or more of: a medical imaging system 240 such as one of the example medical imaging systems described above; a patient bed 250; an injector (not illustrated in Fig. 2) for injecting a contrast agent into the vasculature; and a user input device (not illustrated in Fig. 2) configured to receive user input in relation to the operations performed by the one or more processors 220, such as a keyboard, a mouse, a touchscreen, and so forth.

The above examples are to be understood as illustrative of the present disclosure, and not restrictive. Further examples are also contemplated. For instance, the examples described in relation to a computer-implemented method, may also be provided by the computer program product, or by the computer-readable storage medium, or by the system 200, in a corresponding manner. It is also noted that in the system 200, a single unit or device may fulfil the functions of multiple items recited in the claims. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of predicting vessel characteristics, the method comprising:
receiving (S110) angiographic data comprising an extracorporeal image (110) representing a vessel (120);
inputting (S120) the extracorporeal image (110) into a neural network (130);
generating (S130) one or more predicted vessel characteristics (140) for the vessel (120) using the neural network (130) in response to the inputting; and
outputting (S140) the one or more predicted vessel characteristics (140); and
wherein the neural network (130) is trained to generate the one or more predicted vessel characteristics (140) from the inputted extracorporeal image (110) using training data comprising a plurality of extracorporeal training images (150) representing the vessel (120), and for each extracorporeal training image, an intravascular training image (160) representing the vessel (120), and ground truth data corresponding to the training data, the ground truth data representing the one or more vessel characteristics (140).

2. The computer-implemented method according to claim 1, wherein for each of a plurality of the intravascular training images, the intravascular training image (160) and the corresponding extracorporeal training image (150) are mutually registered.

3. The computer-implemented method according to claim 1 or claim 2, wherein the neural network (130) is trained to generate the one or more predicted vessel characteristics (140) by:
inputting the extracorporeal training images (150) into a first generator neural network (170); and
generating a virtual intravascular training image (160^{V}) corresponding to each extracorporeal training image using the first generator neural network (170) in response to the inputting;
wherein the first generator neural network (170) is trained to generate the virtual intravascular training images (160^{V}) using second training data comprising a plurality of extracorporeal training images (150) representing the vessel (120), and for each extracorporeal training image, a corresponding intravascular training image (160) representing the vessel (120); and
inputting the intravascular training images (160) into a second generator neural network (180); and
generating a virtual extracorporeal training image (150^{V}) corresponding to each intravascular training image using the second generator neural network (180) in response to the inputting;
wherein the second generator neural network (180) is trained to generate the virtual extracorporeal training images (150^{V}) using third training data comprising a plurality of intravascular training images (160) representing the vessel (120), and for each intravascular training image, a corresponding extracorporeal training image (150) representing the vessel (120); and
training the neural network (130) to generate the one or more vessel characteristics (140) using the virtual intravascular training images (160^{V}) and the virtual extracorporeal training images (150^{V}).

4. The computer-implemented method according to claim 3, wherein the extracorporeal image (110) representing the vessel (120) is a pre-interventional procedure image; and
wherein the method further comprises:
receiving intravascular image data comprising a post-interventional procedure intravascular image representing the vessel (120);
inputting the post-interventional procedure intravascular image into the second generator neural network (180); and
generating a virtual extracorporeal image (150^{V}) corresponding to the inputted post-interventional procedure intravascular image using the second generator neural network (180) in response to the inputting; and
outputting the virtual extracorporeal image (150^{V}).

5. The computer-implemented method according to claim 4, wherein the method further comprises:
comparing the virtual extracorporeal image (150^{V}) with the inputted pre-interventional procedure extracorporeal image (110) representing the vessel (120); and
outputting a result of the comparing.

6. The computer-implemented method according to any previous claim, wherein the outputting (S140) the one or more predicted vessel characteristics (140) comprises:
outputting the one or more predicted vessel characteristics (140) in the form of a list of one or more values, and wherein the one or more values represent the one or more characteristics;
or
outputting the one or more predicted vessel characteristics (140) in the form of a graphical representation of the vessel (120), and wherein the one or more characteristics (140) are annotated on the graphical representation of the vessel.

7. The computer-implemented method according to claim 6, wherein the graphical representation of the vessel (120) comprises a virtual extracorporeal image and/or a virtual intravascular image.

8. The computer-implemented method according to any previous claim, wherein the method further comprises outputting a graphical representation of the corresponding inputted extracorporeal image (110).

9. The computer-implemented method according to any previous claim, wherein the neural network (130) is further trained to generate a confidence measure for the one or more predicted vessel characteristics (140); and
wherein the method further comprises:
generating a value for the confidence measure in response to the inputting; and
outputting the value of the confidence measure.

10. The computer-implemented method according to any previous claim, wherein the ground truth data comprises annotations representing the one or more vessel characteristics, and wherein the annotations are provided on the extracorporeal training images (150) and/or on the intravascular training images (160).

11. The computer-implemented method according to any previous claim, wherein the method further comprises receiving point spread function data representing a point spread function of the inputted extracorporeal image (110); and
i) inputting the point spread function data into the neural network (130); and
generating one or more predicted vessel characteristics (140) for the vessel (120) based further on the point spread function data; and
wherein the training data further comprises point spread function training data representing a point spread function of the extracorporeal training images (150);
or
ii) wherein the outputting (S140) the one or more predicted vessel characteristics (140) comprises outputting the one or more predicted vessel characteristics in the form of a virtual extracorporeal image of the vessel (120), and wherein the one or more characteristics are annotated on the virtual extracorporeal image of the vessel (120); and
wherein the method further comprises processing the virtual extracorporeal image of the vessel (120) using the point spread function to improve an image quality of the virtual extracorporeal image.

12. The computer-implemented method according to any one of claims 1 - 11, wherein the neural network (130) is trained to generate the one or more predicted vessel characteristics (140) by:
receiving the training data; and
for each of a plurality of the extracorporeal training images (150):
inputting the extracorporeal training image, and the corresponding intravascular training image (160), into the neural network (130);
generating one or more predicted vessel characteristics (140) for the vessel (120), using the neural network (130); and
adjusting parameters of the neural network (130) based on a difference between the one or more predicted vessel characteristics (140) and the corresponding one or more vessel characteristics from the ground truth data; and
repeating the inputting, the generating, and the adjusting, until a stopping criterion is met.

13. The computer-implemented method according to claim 12, wherein for each of a plurality of the extracorporeal training images (150), the extracorporeal training image comprises an identification of a location of one or more image artifacts; and
wherein the neural network (130) is trained to generate the one or more predicted vessel characteristics (140) by outputting the one or more predicted vessel characteristics (140) in the form of a graphical representation of the vessel (120), and wherein the one or more characteristics are annotated on the graphical representation of the vessel (120); and
wherein the adjusting parameters of the neural network (130) is performed based on a value of a cost function, and wherein the value of the cost function is determined based on the difference between the one or more predicted vessel characteristics (140) and the corresponding one or more vessel characteristics from the ground truth data, and wherein the value of the cost function is penalized based on a presence of an image artifact in the corresponding location in the graphical representation of the vessel (120).

14. A computer program product comprising instructions which when executed by one or more processors (210), cause the one or more processors to carry out the method according to any one of claims 1-13.

15. A system (200) for predicting vessel characteristics, the system comprising:
an input unit (210);
one or more processors (220); and
an output unit (230);
wherein the input unit is configured to:
receive (S110) angiographic data comprising an extracorporeal image (110) representing a vessel (120); and
provide the angiographic data to the one or more processors (210);
wherein the one or more processors (210) are configured to:
implement a neural network (130);
input (S120) the extracorporeal image (110) into the neural network (130); and
generate (S130) one or more predicted vessel characteristics (140) for the vessel (120) using the neural network (130) in response to the inputting; and
wherein the output unit (220) is configured to output (S140) the one or more predicted vessel characteristics (140); and
wherein the neural network (130) is trained to generate the one or more predicted vessel characteristics (140) from the inputted extracorporeal image (110) using training data comprising a plurality of extracorporeal training images (150) representing the vessel (120), and for each extracorporeal training image, an intravascular training image (160) representing the vessel (120), and ground truth data corresponding to the training data, the ground truth data representing the one or more vessel characteristics (140).
